(19) Europäisches Patentamt
European Patent Office
Office européen des brevets

(11) EP 0 914 606 B1

(12) # EUROPÄISCHE PATENTSCHRIFT

(45) Veröffentlichungstag und Bekanntmachung des Hinweises auf die Patenterteilung:
**21.08.2002 Patentblatt 2002/34**

(21) Anmeldenummer: **97935426.3**

(22) Anmeldetag: **16.07.1997**

(51) Int Cl.$^7$: **G01N 33/00**, G01N 33/497

(86) Internationale Anmeldenummer:
**PCT/DE97/01494**

(87) Internationale Veröffentlichungsnummer:
**WO 98/003867 (29.01.1998 Gazette 1998/04)**

(54) **ANALYSEVERFAHREN ZUR SCHNELLEN UND HOCHEMPFINDLICHEN REGISTRIERUNG VON DUFTSTOFFGEMISCHEN IN DER ATMOSPHÄRE**

FAST AND HIGHLY SENSITIVE RECORDING PROCESS FOR ODORIFEROUS SUBSTANCES IN THE ATMOSPHERE

PROCEDE D'ENREGISTREMENT RAPIDE ET DE HAUTE PRECISION DE MELANGES DE SUBSTANCES ODORANTES DANS L'ATMOSPHERE

(84) Benannte Vertragsstaaten:
**AT BE CH DE FR GB LI NL**

(30) Priorität: **20.07.1996 DE 19629338**

(43) Veröffentlichungstag der Anmeldung:
**12.05.1999 Patentblatt 1999/19**

(73) Patentinhaber: **Forschungszentrum Jülich GmbH
52425 Jülich (DE)**

(72) Erfinder:
- **SCHÖNING, Michael
D-52428 Jülich (DE)**
- **KOCH, Uwe, T.
D-67737 Olsbrücken (DE)**
- **SCHÜTZ, Stefan
D-35392 Giessen (DE)**

(74) Vertreter: **Möbus, Steffen
Kayser & Möbus
Patentanwälte
Leienfelsstrasse 6
81243 München (DE)**

(56) Entgegenhaltungen:
**DE-A- 3 906 004**
**NL-A- 9 302 287**
**US-A- 4 142 860**
**US-A- 5 325 867**

- **S. SCHÜTZ, ET AL.: "BIOSENSOR FOR PLANT DAMAGES BY INSECTS" MED. FAC. LANDBOUWW. UNIV. GENT, Bd. 59, Nr. 2B, 1994, GENT, BE, Seiten 691-699, XP000646209**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur Bestimmung von Duftstoffkonzentrationen in der Umgebungsluft mittels Elektroantennogrammen gemäß Anspruch 1.

Die Untersuchung von Duftstoffgemischen niedriger Konzentration in der Atmosphäre ist in Verbindung mit vielen Fragestellungen von Bedeutung. Ein Beispiel ist die Untersuchung der Pflanzenduftstoffe, die von Kulturpflanzen (z.B. Kartoffeln) abgegeben werden und dazu benutzt werden könnten, einen Befall durch Fraßinsekten (Kartoffelkäfer) zu diagnostizieren.

Mit schnellen Detektoren für spezifische Pflanzenduftstoffe kann in Kombination mit schnellen Windmessungen der Gesamtfluß dieser Stoffe in die Atmosphäre bestimmt werden. Dies ist besonders wichtig für die Analyse von Stoffaustausch-Vorgängen in der Atmosphäre.

Aus der DE 39 06 004 A1 ist der schnelle Nachweis von Duftstoffen in der Atmosphäre im ppbv-Bereich durch den Einsatz von Freiland-Elektroantennogrammen (EAG) bekannt. Diese wurden bisher jedoch nur zum Nachweis von Pheromonen benutzt.

Aus J. Insect. Physiol., Vol. 30 (1984) Seite 507-510 ist ein Verfahren zur Identifikation unterschiedlicher Chemorezeptoren durch eine Elektro-Antennogrnmmaufreichnung bekannt. Es werden Untersuchungen mit ein, zwei und sogar drei Duftstoffen beschrieben. Rückschlüsse für ein Analyseverfahren, das zum Nachweisen von beliebigen Duftstoffen geeignet ist, werden aber nicht getroffen.

Aus Entomologia Experimentalis et Applicata, Vol. 77 (1995) Seite 37-46 wird ebenfalls ein Verfahren beschrieben, in welchem mittels Elektro-Antennogrammen verschiedene Stoffgruppen nachgewiesen werden. Auch hier richten sich die Untersuchungen auf ganz spezielle Elektro-Antennogramme und werden keine Rückschlüsse für allgemeines Analyseverfahren beschrieben, das zum Nachweisen von beliebigen Duftstoffen geeignet ist.

Aus CEC/IOBC Symposium, Athens, Nov. 1982, Seite 248-251 ist die Verwendung eines Fruchtfliegen-Elektro-Antennogramms zum Nachweis von Duftstoffen von Früchten beschrieben. Auch dieses Verfahren gibt keine Rückschlüsse auf ein allgemein gültiges Analyseverfahren, das zum Nachweisen beliebiger Duftstoffe geeignet ist.

Die genannten Verfahren des Standes der Technik können als Grundlage für die "Eichung" und Kalibrierung von Insektenantennen zu bestimmten Duftstoffen angesehen werden.

Aus J. Insect. Physiol. Vol. 41 (1995) Seite 465-471 wird ebenfalls ein Verfahren zum Nachweis von Pheromonkonzentrationen unter Verwendung von Elektro-Antennogrammen beschrieben. In diesem Stand der Technik wird sogar festgestellt, daß die EAG-Amplituden als Antwort auf verschiedene Reize (Pheromone und Umgebungsdüfte) nicht unabhängig voneinander sind.

Der Erfindung liegt somit die Aufgabe zugrunde, ein Verfahren zur Bestimmung von Duftstoffkonzentrationen in der Umgebungsluft mittels Elektro-Antennogrammen zu schaffen, mit denen auch leicht zerfallende Stoffgruppen (z.B. Aldehyde, Alkohole und Terpene) atmosphärischer Duftstoffgemische im Konzentrationsbereich pptv nachzuweisen sind, die mit einer Zeitauflösung im Sekundenbereich arbeiten, leicht handhabbar und kostengünstig sind.

Diese Aufgabe wird erfindungsgemäß durch ein Verfahren mit den Merkmalen des Anspruchs 1 gelöst.

Das erfindungsgemäße neue Meßverfahren macht sich die Tatsache zunutze, daß eine Insektenantenne mit unterschiedlichen Rezeptorklassen besetzt ist, die jeweils auf unterschiedliche Stoffgruppen selektiv antworten.

Durch das erfindungsgemäße Meßverfahren können mit einer Insektenantenne komplexe Duftstoffgemische schnell und genau erfaßt und analysiert werden.

Weitere Vorteile des Verfahrens ergeben sich aus den Merkmalen der Unteransprüche.

Das Verfahren der vorliegenden Erfindung wird im folgenden am Beispiel anhand der Zeichnungen näher beschrieben. Diese zeigen eine Anordnung , die bei der Ausöführung des Verfahrens verwendet wird. Es zeigen:

Fig. 1 eine schematische Darstellung in Seitenansicht der Anordnung in Seitenansicht;
Fig. 2eine schematische Darstellung der Anordnung aus Fig. 1 in Draufsicht.

In Fig. 1 ist die Anordnung zur Registrierung und Messung eines multidimensionalen Elektroantennogramms (MD-EAG) schematisch dargestellt. Die Insektenantenne wird in einer Halterung 1, z. B. aus Kunststoff, erschütterungssicher gelagert. Die Halterung 1 ist in einer Kapsel 2 am Ende eines Hauptluftrohres 3 montiert. Durch das Hauptluftrohr 3 wird mittels einer kleinen Saugluftpumpe 4 ein ständiger Luftstrom (Hauptluftstrom) geleitet. Der Einlaß für den Hauptluftstrom im Hauptluftrohr 3 ist durch ein Aktivkohlefilter 5 verschlossen, so daß nur gereinigte Luft zur Insektenantenne gelangt.

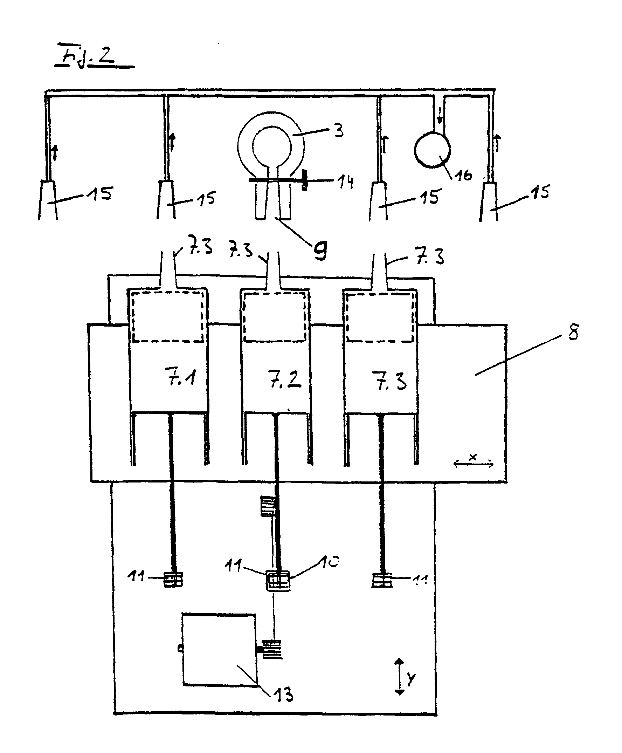

Eine Kalibriereinheit 7 mit Dosierpumpen 7.1, 7.2, und 7.3 (siehe Fig. 2) sind seitlich vom Hauptluftrohr 3 angebracht. Die Dosierpumpen 7.1, 7.2, 7.3 umfassen jeweils einen Zylinder 7a, in welchem eine kleine Menge Öl mit einem darin verdünnt enthaltenden Duftstoff vorhanden ist. Die Kalibriereinheit 7 ist auf wenigstens einem Auflagetisch 8 angeordnet Für die Kalibrier- und Zusatzreize ist die Kalibriereinheit 7 auf wenigstens einer Seite der des Hauptluftrohrs 3 angeordnet (in Fig. 1 auf beiden Seiten).

Bei der bisherigen Verwendung der Freiland-EAG's waren nur 3 Dosierpumpen sternfbrmig um das Hauptluftrohr 3 herum angebracht. Beim Betrieb des MD-EAG sind jedoch für jeden Basisreizstoff mindestens zwei Kalibrierquellen, d. h., Dosierpumpen 7.1, 7.2, 7.3 mit unterschiedlichen Verdünnungsstufen erforderlich.

In der beschriebenen Anordnung sind die benötigten Dosierpumpen 7.1, 7.2, 7.3 nebeneinander auf dem Auflagetisch 8 angeordnet und dort durch geeignete Halteelemente (nicht dargestellt) festgelegt. Der Auflagetisch 8 kann über Schrittmotoren (nicht dargestellt) kontrolliert in *x*- und *y*-Richtung verschoben werden. Um einen vorgegebenen Kalibrierreiz zu setzen, wird der Auflagetisch 8 in *x*-Richtung verfahren, bis die gewünschte Dosierpumpe 7.2 (Fig. 2) mit ihrer Ausgangsöffnung 7.4 mit einem Einschußloch 9 am Hauptluftrohr 3 ausgerichtet ist. Über einen elektrisch betätigten Andruckmagnet 10 (Fig.2) wird ein Mitnehmer 11 auf einer Schubstange 12 mit einem Schrittmotor 13 gekoppelt. Dann wird der Auflagetisch 8 in *y*-Richtung verfahren, bis die Dosierpumpe 7.2 in das Hauptluftrohr 3 hineinragt, und die Dosierpumpe 7.2 mit dem Schrittmotor 13 aktiviert werden kann.

Soll eine andere Dosierpumpe 7.1 oder 7.3 benutzt werden, so wird zunächst der Schrittmotor 13 in die Ausgangsstellung zurückgefahren und der Andruckmagnet 10 gelöst. Dann wird der Auflagetisch 8 in der *y*-Richtung in die Ausgangsstellung zurückgefahren und damit die Dosierpumpe 7 aus dem Hauptluftrohr 3 gezogen. Ein Ventil 14 verschließt den Anschluß zum Hauptluftstrom in den Zeiten, in denen keine Dosierpumpe 7.1, 7.2, 7.3 am Hauptluftrohr 3 angekoppelt ist.

Die Steuerung der hier beschriebenen Abläufe kann über einen Microcontroller (nicht dargestellt) geschehen, wodurch die Zeiten zwischen den einzelnen Kalibrierreizen klein gehalten werden können. Eine Reihe von Aufnahmekanälen 15 nimmt in der vorgefahrenen Stellung des Auflagetisches 8 die einzelnen Ausgangsöffnungen 7.4 der Dosierpumpen 7.1, 7.2, 7.3 auf und sorgt mit einer schwachen Absaugströmung (Pumpe 16) dafür, daß keine Reizstoffe aus den Dosierpumpen 7.1, 7.2, 7.3 in die Außenluft gelangen und die Messungen verfälschen können.

Die Steuerung, z. B. mittels Microcontroller, ist allgemein bekannt und soll hier nicht weiter beschrieben werden. Die Schrittmotoren für die Verstellung des Auflagetisches 8 in *x*-Richtung, die Schrittmotoren 13 für die Verstellung in *y*-Richtung sowie die entsprechenden konstruktiven Verbindungselemente sind an sich bekannte Regelungselemente und werden in den Ansprüchen unter dem Oberbegriff "Regelungseinrichtung" zusammengefaßt.

Die erfmdungsgemäße Ausbildung der MD-EAG-Apparatur mit dem Auflagetisch 8 vermeidet die sonst nötige große Anzahl von Schrittmotoren 13 sowie den damit verbundenen konstruktiven Aufwand und ermöglicht die Verwendung vieler Dosierpumpen, ohne das Gewicht der Meßsonde wesentlich zu erhöhen (Fig. 2 ).

Auch wenn in Fig. 2 nur drei Dosierpumpen 7.1, 7.2, 7.3 dargestellt sind, so ist dies so zu verstehen, daß selbstverständlich jede andere beliebige Zahl von Dosierpumpen entsprechend angeordnet und gesteuert werden kann. Die Anzahl wird alleine vom Bedarf bestimmt.

Das Meßprinzip des "multidimensionalen EAG" (MD-EAG) läuft in 3 Phasen ab:

**1. Phase:** Für eine bestehende Meßaufgabe wird die Antenne einer Insektenart ausgewählt, die Rezeptoren für den Bereich der zu untersuchenden Stoffklassen besitzt. Sodann werden für diesen Antennentyp Basisreizstoffe ($b_i$) ($i$ = 1...$n$) ermittelt, wobei für jeden Basisreizstoff gelten soll:

1) er löst eine starke EAG-Reaktion aus;

2) die EAG-Antwort auf einen Reiz mit dem Basisstoff $b_i$ zeigt die gleiche Amplitude, auch wenn die Antenne gleichzeitig mit einem anderen Basisreizstoff $b_k$ ($k$ = 1...$n$, $k \neq i$) als Dauerreiz gereizt wird.

Die Eigenschaft 2) soll als "orthogonal" bezeichnet werden.

Ein Beispiel für zwei orthogonale Reizstoffklassen sind Pheromone und Pflanzenduftstoffe, z.B. bei der Antenne des bekreuzten Traubenwicklers. Die Bestimmung eines solchen Satzes orthogonaler Basisreizstoffe muß für eine Antennenart nur einmal erfolgen. Für eine konkrete Messung mit dem MD-EAG wird für jeden Basisreizstoff je ein Satz von Kalibrierquellen benötigt, z.B. drei Dosierpumpen 7.1, 7.2, 7.3 die mit einer Reizstoff-Öl-Mischung 6 bestückt sind. Es wurde festgestellt, daß durch unterschiedliche Konzentrationen des Reizstoffs im Öl unterschiedliche Reizstoffkonzentrationen in der Luft hergestellt werden können.

**2. Phase:** Im Meßsystem (siehe unten) wird eine gegebene Antennenpräparation kalibriert. Dazu wird die Antenne in gereinigter Luft nacheinander den verschiedenen Konzentrationen der Kalibrierquellen ausgesetzt, und so die Empfindlichkeit $c_i$ und Nachweisschwelle $b_{0i}$ der Antenne für jeden Basisreizstoff $b_i$ bestimmt.

**3. Phase:** Zur Analyse eines Duftgemisches in der Atmosphäre wird nun das Luftfilter am Eingangsrohr entfernt. Während die Außenluft ständig zur Antenne geleitet wird, wird jeweils ein kurzer Impuls der verschiedenen Kalibrierquellen in angemessener Konzentration hinzugegeben. Aus der durch den Zusatzreiz mit dem Basisreizstoff $b_i$ erzeugten Zusatz-Amplitude $\Delta A_{im}$ kann dann die Konxentration der Duftstoffe in der Stoffklasse i bestimmt werden.

Dabei gilt: Große Zusatzamplitude bei Zusatzreiz $b_i$ bedeutet kleine Konzentration in der Stoffklasse *i* und umgekehrt.

So läßt sich jedem Außenluft-Gemisch ein "Duftvektor" (1....*n*) zuordnen, dessen Komponenten als "Duftkomponenten der Klasse *i*" bezeichnet werden.

Duftgemische gleicher Zusammensetzung, aber verschiedener Konzentrationen erscheinen dann als eine Längendehnung bzw. Kürzung des gleichen Duftvektors ohne Änderung der Richtung.

Die mathematische Formulierung des Meßverfahrens ist wie folgt:

Zwischen einem Reiz mit dem Stoff $b_i$ in der Konzentration *i* und der Antwort-Amplitude $A_i$ sei ein funktionaler Zusammenhang (Reiz-Antwort-Funktion) der allgemeinen Form

$$A_i = Q_i(\alpha_i \cdot b_i)$$

gegeben.

Sind die Konzentrationen der Basisreizstoffe $b_i$ in den Dosierpumppen (Verdünnungsstufe m) mit $b_{im}$ gegeben, so folgt für die Antwort-Amplitude $A_{im}$ des EAG auf einen Kalibrierreiz $A_{im} = Q_i(\nu \cdot b_{im})$ mit dem Verdünnungsfaktor

$$\nu = \frac{Kalibrierluftstrom}{Hauptluftstrom}.$$

Zunächst sei angenommen, daß in der Außenluft nur Reizstoffe enthalten sind, die sich aus unterschiedlichen Konzentrationsfaktoren *i* der Basisreizstoffe $b_i$ zusammensetzen. Dann gilt wegen der Orthogonalität der Basisreizstoffe für die Plateau-Amplitude der MD-EAG-Antwort bei Öffnung des Kohlefilters

$$A_F = \sum_{i=1}^{n} Q_i(\alpha_i \cdot b_i).$$

Wird nun bei offenem Filter ein Zusatzreiz mit der Kalibrierquelle $b_{km}$ gegeben, so erhält man für die Gesamt-Amplitude der MD-Antwort:

$$A_F^{+km} = Q_k(\nu \cdot b_{km} + w \cdot \alpha_k \cdot b_k) + \sum_{\substack{i=1 \\ i \neq k}}^{n} Q_i(w \cdot \alpha_i \cdot b_i) \qquad \text{mit } w = 1 - \nu.$$

Für die Zusatzamplitude $\Delta A_{km}$ ergibt sich

$$\Delta A_{km} = A_F^{+km} - A_F = Q_k(\nu \cdot b_{km} + w \cdot \alpha_k \cdot b_k) + \sum_{\substack{i=1 \\ i \neq k}}^{n} Q_i(w \cdot \alpha_i \cdot b_i) - \sum_{i=1}^{n} Q_i(\alpha_i \cdot b_i).$$

Durch Umformen erhält man daraus

$$\text{(Gl. 1)}\ \Delta A_{km} = Q_k(\nu \cdot b_{km} + w \cdot \alpha_k \cdot b_k) - Q_k(\alpha_k \cdot b_k) + \sum_{\substack{i=1 \\ i \neq k}}^{n} \left(Q_i(w \cdot \alpha_i \cdot b_i) - Q_i(\alpha_i \cdot b_i)\right).$$

Für die Reiz-Antwort-Funktionen der Basisreizstoffe $b_i$ wird der Ansatz

$$\text{(Gl. 2)} \qquad Q_i(b_i) = c_i \cdot log(b_i + b_{0i}) + d_i$$

für alle $i$ gemacht, wobei $c_i$ die Empfindlichkeit der Antenne und $b_{0i}$ die Nachweisschwelle für den Stoff $b_i$ ist. Die Konstante $d_i$ sei so definiert, daß $Q_i(b_i) = 0$ für $b_i = 0$ ist.

Durch Einsetzen von Gleichung 2 in Gleichung 1 ergibt sich Gleichung 3.

(Gl. 3)
$$\Delta A_{km} = c_k \cdot \log(v \cdot b_{km} + w \cdot \alpha_k \cdot b_k + b_{0k}) + d_k - (c_k \cdot \log(\alpha_k \cdot b_k + b_{0k}) + d_k) + \sum_{\substack{i=1 \\ i \neq k}}^{n} \left(c_i \cdot \log(w \cdot \alpha_i \cdot b_i + b_{0i}) + d_i - (c_i \cdot \log(\alpha_i \cdot b_i + b_{0i}) + d_i)\right)$$

Durch Umformen ergibt sich daraus

$$\alpha_k \cdot b_k = \frac{v \cdot b_{km}}{F - w} - b_{0k} \cdot \frac{F - 1}{F - w}$$

wobei

$$F = 10^{\left(\frac{\Delta A_{km}}{c_k} + \sum_{\substack{i=1 \\ i \neq k}}^{n} \frac{c_i}{c_k} \log\left(\frac{w \cdot \alpha_i \cdot b_i + b_{0i}}{\alpha_i \cdot b_i + b_{0i}}\right)\right)}$$

ist.

Für $F \gg 1$ und $w \approx 1$ kann $\frac{F - 1}{F - w} = 1$ gesetzt werden und man erhält nach weiteren Umformungen

(Gl. 4)
$$\alpha_k \cdot b_k = \frac{v \cdot b_{km}}{10^{\frac{\Delta A_{km}}{c_k}} \cdot \underbrace{\prod_{\substack{i=1 \\ i \neq k}}^{n} \left(\frac{w \cdot \alpha_i \cdot b_i + b_{0i}}{\alpha_i \cdot b_i + b_{0i}}\right)^{\frac{c_i}{c_k}}}_{g} - w} + b_{0k}$$

Aus Gleichung 4 können die Koeffizienten $i$ der Reihe nach bestimmt werden, da die $c_i$ und $b_{0i}$ zuvor in der Kalibrierphase bestimmt wurden. Zunächst wird dabei die Größe $g = 1$ gesetzt, wodurch sich die $i$ näherungsweise berechnen lassen. In einem zweiten Schritt können dann die genäherten Werte $i$ zur Berechnung von $g$ eingesetzt werden, und man bekommt korrigierte Werte für die $i$, wodurch der Duftvektor $\{\alpha_1 ... \alpha_n\}$ genau bestimmt werden kann. Dieses Verfahren funktioniert ebenso, wenn in der Außenluft Duftstoffe existieren, die gleichzeitig mehrere Rezeptorklassen der Antenne reizen.

Durch die selektive Reizung mit den Zusatzreizen durch die orthogonalen Basisreizstoffe wird jeweils die Besetzung einer Rezeptorklasse selektiv ausgemessen, so daß die anteilige Wirkung eines unbekannten Duftstoffs auf die jeweilige Rezeptorklasse bestimmt werden kann. Das System ist somit in der Lage, völlig unbekannte Duftstoffe zu klassifizieren, sofern sie überhaupt eine Reizung der Antenne bewirken.

Zusammengefaßt läßt sich der Erfindungsgedanke wie folgt wiedergeben:

1) Es ist eine Anordnung zur Erzeugung von reproduzierbaren Gemischen aus Luft und Duftstoffen zur Kalibrierung von Freiland-EAG-Meßsystemen vorgesehen, die Dosierpumpen 7.1, 7.2, 7.3 aufweist, die mit einer kleinen Menge Öl 6 gefüllt sind, in dem der Duftstoff verdünnt enthalten ist, deren Kolben 7a über Schrittmotoren 13 bewegt werden.

2) Diese Anordnung umfaßt eine große Zahl von Dosierpumpen, die auf einem Auflagetisch 8 gelagert sind. Durch motorische Antriebe wird wenigstens eine der Dosierpumpen am EAG-Meßsystem angeschlossen.

3) Diese Anordnung umfaßt zudem eine Kupplungsvorrichtung 9, 10, die für jeden Dosierpumpensatz einen gemeinsamen Antrieb mit nur einem Schrittmotor 13 ermöglicht.

4) Ein Meßverfahren, mit dem unter Verwendung dieser Anordnung für Antennen einer bestimmten Insektenart in einer EAG-Apparatur typische Reizstoffe (Basisreizstoffe) selektiert werden können, die einzelne Rezeptorklassen der Antenne selektiv reizen.

5) Ein Meßverfahren, das die mit dem vorstehenden Verfahren gewonnenen Basisreizstoffe in einer Freiland-EAG-Meßanordnung unter Nutzung einer erfindungsgemäßen Kalibriereinheit nutzt, um Duftstoffgemische zu charakterisieren.

6) Ein Rechenverfahren, das aus den Meßverfahren gewonnenen EAG-Daten durch eine quantitative numerische Berechnung Duftstoff-Konzentrationswerte bestimmt.

## Patentansprüche

**1.** Verfahren zur Bestimmung von Duftstoffkonzentrationen in der Umgebungsluft mittels Elektroantennogrammen, in welchem eine Antenne einer Insektenart ausgewählt wird, die mit Rezeptoren besetzt ist, von denen wenigstens ein Teil auf den nachzuweisenden Duftstoff anspricht, wobei für die ausgewählte Insektenantenne ein für den Duftstoff charakterisierender Basisreizstoff ($b_i$) ermittelt wird und die ausgewählte Insektenantenne in gereinigter Luft kalibriert wird, wobei Basisreizstoffe ($b_i$) für die nachzuweisenden Duftstoffe vor dem Einsatz der Insektenantenne in Abhängigkeit von zwei Eigenschaften ermittelt werden:

- 1) sie lösen eine starke Elektroantennogramm-Reaktion ($A_i$) aus;
- 2) die Elektroantennogramm-Reaktion auf einen Reiz mit einem der Basisreizstoffe ($b_i$) zeigt eine unveränderte Amplitude ($A_i$), auch wenn die Antenne gleichzeitig mit einem anderen Basisreizstoff ($b_k$) als Dauerreiz gereizt wird;
- wobei die Antenne zur Kalibrierung nacheinander verschiedenen Konzentrationen der Basisreizstoffe ($b_i$) ausgesetzt und deren Empfindlichkeit auf den jeweiligen Basisreizstoff ($b_i$) bestimmt wird,
- wobei die ausgewählte Insektenantenne nach der Kalibrierung während ihres Einsatzes in der Umgebungsluft kurzen Impulsen der Basisreizstoffe ($b_i$) ausgesetzt wird, und
- wobei aus den durch die Zusatzreizstoffe mit den Basisreizstoffen ($b_i$) erzeugten Zusatzamplituden ($\Delta A_i$) zu der in der Umgebungsluft gemessenen Amplitude ($A_F$) die Konzentration der Duftstoffe bestimmt wird.

**2.** Verfahren nach Anspruch 1,
**dadurch gekennzeichnet,**
**daß** durch die Kalibrierung in gereinigter Luft die Nachweisschwelle der Insektenantenne für jeden Basisreizstoff ($b_i$) bestimmt wird.

**3.** Verfahren nach Anspruch 1 oder 2,
**dadurch gekennzeichnet,**
**daß** für die Bestimmung der Konzentration der Duftstoffe eine quantitative numerische Berechnung durchgeführt wird.

## Claims

**1.** A method for determining concentrations of aromatic substances in the ambient air by means of electroantennograms, wherein the antenna of an insect species is selected which contains receptors, of which at least a part responds to the aromatic substance to be detected, wherein a basic stimulant ($b_i$) that characterizes the aromatic substance is determined for the selected insect antenna and the selected insect antenna is calibrated in cleaned air, and wherein basic stimulants ($b_i$) for the aromatic substances to be detected are determined in dependence on two properties before the insect antenna is used:

- 1) they trigger a strong electroantennogram reaction ($A_i$), and
- 2) the electroantennogram reaction to a stimulation with one of the basic stimulants ($b_i$) shows an unchanged amplitude ($A_i$), namely even if the antenna is simultaneously stimulated with another basic stimulant ($b_k$) in the form of a permanent stimulation,
- wherein the antenna is successively subjected to different concentrations of the basic stimulants ($b_i$) and its sensitivity to the respective basic stimulant ($b_i$) is determined in order to calibrate the antenna,
- wherein the selected insect antenna is, after the calibration, subjected to brief pulses of the basic stimulants ($b_i$) during its use in the ambient air, and
- wherein the concentration of the aromatic substances is determined from the amplitudes ($\Delta A_i$) generated in addition to the amplitude ($A_F$) measured in the ambient air by the stimulants present in addition to the basic stimulants ($b_i$).

**2.** The method according to Claim 1, **characterized by** the fact that the detection threshold of the insect antenna for each basic stimulant ($b_i$) is determined by the calibration in cleaned air.

**3.** The method according to Claim 1 or 2, **characterized by** the fact that a quantitative numerical calculation is carried out in order to determine the concentration of the aromatic substances.

## Revendications

**1.** Procédé pour déterminer les concentrations de parfum dans l'air ambiant au moyen de diagrammes d'antenne électrique, avec lequel on sélectionne une antenne d'une espèce d'insecte qui est équipée de récepteurs, dont au moins une partie réagit au parfum à déceler, moyennant quoi on détermine pour l'antenne d'insecte sélectionnée un agent irritant de base ($b_i$) caractéristique du parfum et on étalonne l'antenne d'insecte sélectionnée dans l'air purifié, les agents irritants de base ($b_i$) pour les parfums à déceler étant déterminés avant l'utilisation de l'antenne d'insecte en fonction de deux propriétés :

- 1) déclenche une forte réaction du diagramme d'antenne électrique ($A_i$) ;
- 2) la réaction du diagramme d'antenne électrique à une irritation avec l'un des agents irritants de base ($b_i$) montre une amplitude ($A_i$) inchangée, même si l'antenne est irritée en même temps avec un autre agent irritant de base ($b_k$) sous la forme d'une irritation permanente ;
- l'antenne étant exposée pour l'étalonnage de façon successive à différentes concentrations successives des agents irritants de base ($b_i$) et sa sensibilité à l'agent irritant de base ($b_i$) concerné étant déterminée,
- l'antenne d'insecte sélectionnée étant exposée après l'étalonnage pendant son utilisation dans l'air ambiant à de brèves impulsions des agents irritants de base ($b_i$), et
- la concentration des parfums étant déterminée à partir des amplitudes supplémentaires ($\Delta A_i$) générées par les agents irritants supplémentaires avec les, agents irritants de base ($b_i$) pour l'amplitude ($A_F$) mesurée dans l'air ambiant.

**2.** Procédé selon la revendication 1, **caractérisé en ce que** le seuil de décèlement de l'antenne d'insecte est déterminé pour chaque agent irritant de base ($b_i$) par l'étalonnage dans l'air purifié.

**3.** Procédé selon la revendication 1 ou 2, **caractérisé en ce que** l'on effectue un calcul numérique quantitatif pour la détermination de la concentration des parfums.

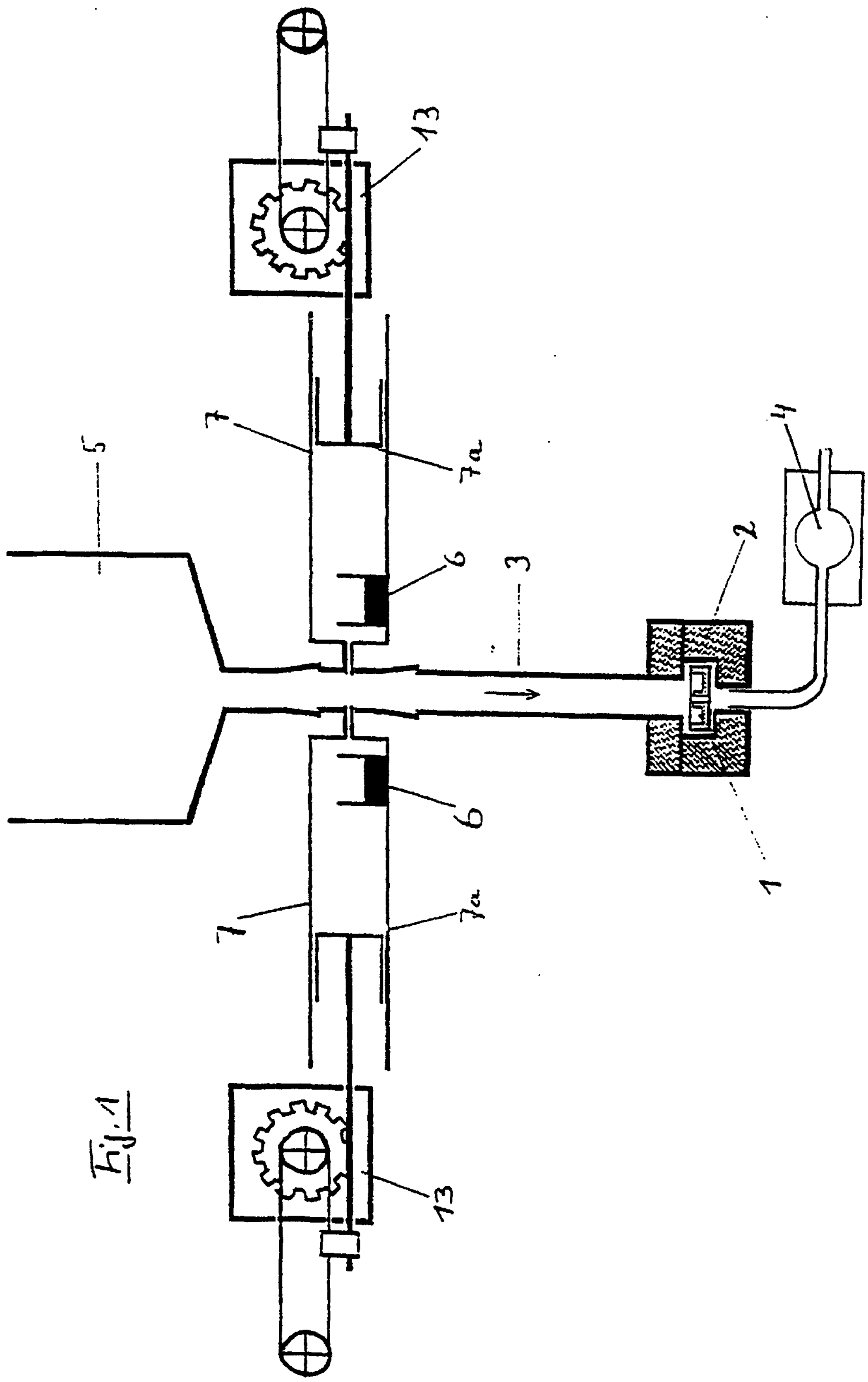


Fig. 1

Fig. 2